# EUROPEAN PATENT SPECIFICATION

(11) **EP 4 226 848 B1**
(45) Date of publication and mention of the grant of the patent: **03.09.2025**
(21) Application number: 22859508.8
(22) Date of filing: 15.09.2022
(51) Int. Cl.: A61B 5/1455

(54) **PPG CONTROL METHOD AND APPARATUS, AND ELECTRONIC DEVICE**
PPG-STEUERUNGSVERFAHREN UND -VORRICHTUNG SOWIE ELEKTRONISCHE VORRICHTUNG
PROCÉDÉ ET APPAREIL DE COMMANDE DE PPG, ET DISPOSITIF ÉLECTRONIQUE

(30) Priority: 30.12.2021 CN 202111654810; 14.01.2022 CN 202210042886
(43) Date of publication of application: 16.08.2023
(73) Proprietor: Honor Device Co., Ltd., Shenzhen, Guangdong 518040 (CN)
(72) Inventor: CAI, Xinpei, Shenzhen, Guangdong 518040 (CN)
(74) Representative: Isarpatent
(86) International application number: PCT/CN2022/118962
(87) International publication number: WO 2023/124252

(56) References cited:
- CN-A- 112 741 626
- CN-A- 112 998 671
- CN-A- 113 440 102
- CN-A- 114 983 353
- US-A1- 2020 054 219

## Description

### TECHNICAL FIELD

This disclosure generally relates to the field of measurement technologies, and the invention in particular, relates to a photoplethysmography (photoplethysmography, PPG) control method, an electronic device, a computer program product and a computer-readable storage medium.

### BACKGROUND

In an application scenario of a conventional technology, wearable products, such as smartwatches, need to use photoplethysmography (photoplethysmography, PPG) to measure human health characteristics such as a heart rate, a respiratory rate, and blood oxygen.

Using a smartwatch as an example, currently, a common PPG implementation method is to arrange a PPG module on the back of the smartwatch. The PPG module includes a light-emitting diode (light-emitting diode, LED) and a photodiode (Photo-Diode, PD). Light emitted by the LED irradiates skin and then is reflected, and the reflected light is collected by the PD, and an optical signal collected by the PD is calculated to measure human body characteristics.

A wearable product is usually powered by a battery. Therefore, to prolong a battery duration of the wearable product, power consumption of the PPG module needs to be minimized. In addition, to obtain accurate human body characteristic parameters as far as possible, it is also required to ensure measurement accuracy of the PPG module on the wearable product. Therefore, a PPG control method is required to reduce the power consumption of the PPG module while ensuring the measurement accuracy of the PPG module.

CN112998671A discloses a PPG signal acquisition method for collecting PPG signals and determining a quality level of the current signal collection environment of the photoelectric sensor. Depending on the quality level of a current signal collection environment, sampling frequency and/or luminous power of the photoelectric sensor is adjusted. Different quality level grades based on the crest coefficients for the quality level of the current signal collection environment are assigned. The proposed method performs pulse rate and blood oxygen measurements and utilizes different auxiliary sensors like pressure sensor or acceleration sensor when determining the quality level of the current signal collection environment.

CN113440102A discloses a sleep apnea monitoring method and an electronic device using different monitoring modes. The disclosed method considers a variable power consumption and can operate in a low-power consumption mode (i.e., single monitoring mode) or in high-power consumption mode (including a confirmation mode and a composite monitoring mode). The electronic device may turn on all or a part of PPG sensors when obtaining heart rate data and/or blood oxygen value data depending on the operation mode. Furthermore, the electronic device includes a sensor module that can comprise a PPG sensor, an acceleration sensor and a motion sensor. The auxiliary sensors like pressure sensor or acceleration sensors are turned on in a composite monitoring mode to acquire and monitor the physiology of a user in a sleep state.

US20200054219A1 discloses a smart wearable device including a detection apparatus that has one set of measuring parts and a plurality of sets of light emitting parts. The device considers heart rate detection and/or blood oxygen detection. The smart wearable device may receive different control instructions. For example, a first control instruction for blood oxygen detection starts the two sets of red/IR LEDs and a second control instruction for heart rate detection starts the one set of green LEDs. Furthermore, three sets of light emitting parts can be three sets of tri-color LEDs of red, IR and green and tri-color LED that may be switched on or off depending on the received control instruction. The electronic device includes an air pressure detection apparatus and a gravitational acceleration sensor but the usage purpose of these auxiliary sensors is not specified.

### SUMMARY

In view of object of how to ensure measurement accuracy of a photoplethysmography (photoplethysmography, PPG) module and reduce power consumption of the PPG module in a conventional technology, the object of the present invention is to provide a PPG control method, an electronic device, a computer program product and a computer-readable storage medium. This object is solved by the attached independent claims and further embodiments and improvements of the invention are listed in the attached dependent claims. Hereinafter, up to the "brief description of the drawings", expressions like "...aspect according to the invention", "according to the invention", or "the present invention", relate to technical teaching of the broadest embodiment as claimed with the independent claims. Expressions like "implementation", "design", "optionally", "preferably", "scenario", "aspect" or similar relate to further embodiments as claimed, and expressions like "example", "...aspect according to an example", "the disclosure describes", or "the disclosure" describe technical teaching which relates to the understanding of the invention or its embodiments, which, however, is not claimed as such.

The following technical solutions are used in embodiments of the invention:

According to a first aspect according to the invention, the invention provides a PPG control method, where the method is applied to an electronic device, and the electronic device includes a PPG module including a light emitting module. The PPG control method according to the invention includes at least the following process steps:
determining a current measurement environment and measurement target, where the measurement environment includes motion, rest, and sleep, and the measurement target includes a heart rate and blood oxygen; and
determining a light emitting mode of the light emitting module either based on the current measurement environment or based on the current measurement environment and the measurement target.

In the method according to this embodiment of the invention, different PPG control schemes are used based on different measurement environments and measurement targets, so that a waste of power can be avoided while measurement accuracy is ensured, thereby reducing power consumption of the PPG module while ensuring measurement accuracy of the PPG module.

In an actual operation scenario, the PPG module may measure a heart rate based on green light, because under the same power consumption, a heart rate measurement effect of green light is better than that of red light, that is, power consumption of emitting green light is less than that of emitting red light when a same measurement effect is achieved.

Therefore, to reduce the power consumption, in a not claimed implementation of the first aspect, the light emitting module includes a first light emitting device, a second light emitting device, and a third light emitting device, where the first light emitting device is configured to emit green light, the second light emitting device is configured to emit red light, and the third light emitting device is configured to emit infrared light. In the execution process of determining the light emitting mode of the light emitting module based on the current measurement environment and measurement target, the light emitting mode of the light emitting module is determined based on the measurement target, and the first light emitting device is enabled when the measurement target is a heart rate; or the second light emitting device and the third light emitting device are enabled when the measurement target is blood oxygen.

In an actual operation scenario, when a same measurement effect is achieved, an LED light intensity required in a motion state is greater than that required in a rest or sleep state.

Therefore, to reduce the power consumption, in an implementation of the first aspect according to the invention, the light emitting module includes a plurality of light emitting devices, and the plurality of light emitting devices support emission of a same type of light. In the execution process of determining the light emitting mode of the light emitting module based on the current measurement environment and measurement target, the light emitting mode of the light emitting module is determined based on the measurement environment, and all light emitting devices of the light emitting module are enabled when the measurement environment is motion; or some light emitting devices of the light emitting module are enabled when the measurement environment is rest or sleep.

In an actual operation scenario, when a user is sleeping, visible light may affect sleep quality of the user.

Therefore, to improve user experience, in an implementation of the first aspect according to the invention, the light emitting module includes a fourth light emitting device and a fifth light emitting device, where the fourth light emitting device is configured to emit visible light, and the fifth light emitting device is configured to emit infrared light. In the process of determining the light emitting mode of the light emitting module based on the current measurement environment and the measurement target, the light emitting mode of the light emitting module is determined based on the measurement environment, and the fourth light emitting device is enabled when the measurement environment is motion or rest; or the fifth light emitting device is enabled when the measurement environment is sleep.

In an actual operation scenario, when blood oxygen is measured, simultaneous emission of red light and infrared light will interfere with each other, which affects measurement accuracy.

Therefore, to improve measurement accuracy, in a not claimed implementation of the first aspect, the light emitting module includes a sixth light emitting device and a seventh light emitting device, where the sixth light emitting device is configured to emit red light, and the seventh light emitting device is configured to emit infrared light. In the process of determining the light emitting mode of the light emitting module based on the current measurement environment and measurement target, the light emitting mode of the light emitting module is determined based on the measurement target, and the sixth light emitting device and the seventh light emitting device are alternately enabled when the measurement target is blood oxygen.

Based on the scheme of controlling the light emitting module according to the first aspect according to the invention, in an implementation of the first aspect according to the invention, the light emitting module includes an eighth light emitting device, a ninth light emitting device, a tenth light emitting device, and an eleventh light emitting device, where the eighth light emitting device and the ninth light emitting device are configured to emit green light, and the tenth light emitting device and the eleventh light emitting device are configured to emit red light and infrared light.

The determining a light emitting mode of the light emitting module based on the current measurement environment and measurement target includes:
enabling the eighth light emitting device and the ninth light emitting device to emit green light when the measurement environment is motion, and the measurement target is a heart rate; or
enabling the eighth light emitting device or the ninth light emitting device to emit green light when the measurement environment is stillness, and the measurement target is a heart rate; or
enabling the tenth light emitting device or the eleventh light emitting device to emit infrared light when the measurement environment is sleep, and the measurement target is a heart rate; or
enabling the tenth light emitting device and the eleventh light emitting device to alternately emit red light and infrared light when the measurement environment is motion, and the measurement target is blood oxygen; or
enabling the tenth light emitting device or the eleventh light emitting device to alternately emit red light and infrared light when the measurement environment is stillness or sleep, and the measurement target is blood oxygen.

In an actual operation scenario, a higher PPG sampling rate indicates higher PPG power consumption.

Therefore, to reduce power consumption, in an implementation of the first aspect according to the invention, the method further includes:
determining a sampling rate of the PPG module based on the current measurement environment, and using a first sampling rate when the measurement environment is motion; or using a second sampling rate when the measurement environment is rest or sleep, where the first sampling rate is greater than the second sampling rate.

Specifically, in an application scenario, the first sampling rate is 100 Hz, and the second sampling rate is 25 Hz.

In an actual operation scenario, a longer PPG integration time indicates higher PPG power consumption.

Therefore, to reduce power consumption, in an implementation of the first aspect according to the invention, the method further includes:
determining an integration time of the PPG module based on the current measurement environment, and using a first integration time when the measurement environment is motion; or using a second integration time when the measurement environment is rest or sleep, where the first integration time is greater than the second integration time.

Specifically, in an application scenario, the first integration time is 79 us, and the second integration time is 39 us.

According to a second but not claimed aspect, the disclosure also provides a PPG control apparatus, where the apparatus is applied to an electronic device, the electronic device includes a PPG module, the PPG module includes a light emitting module, and the apparatus includes:
a collection module, configured to determine a current measurement environment and/or measurement target, where the measurement environment includes motion, rest, and sleep, and the measurement target includes a heart rate and blood oxygen; and
a control module, configured to determine a light emitting mode of the light emitting module based on the current measurement environment and/or measurement target.

According to a third aspect according to the invention, the invention also provides an electronic device, where the electronic device includes a memory configured to store a computer program instruction and a processor configured to execute a computer program instruction, and when the computer program instruction is executed by the processor, the electronic device is triggered to perform the steps of the method according to the first aspect.

According to a fourth aspect according to the invention, the invention also provides a computer-readable storage medium, where the computer-readable storage medium stores a computer program, and when the computer program is run on a computer, the computer is enabled to perform the method according to the first aspect or the second aspect.

In the following description, features which in the above summary of the invention have been marked as "not claimed" or "according to the invention" are also hereinafter, when they are described and explained with reference to the drawings, to be understood as "not claimed" or "not part of the invention" or "according to the invention". Even if sometimes in the description of the embodiments below, features marked above "according to the invention" or "the invention" are referred to in connection with the words "can" or "may" or other expressions which contain the notion of them being "optional", it should be understood that indeed such features are considered essential to the invention as claimed and not optional.

### BRIEF DESCRIPTION OF DRAWINGS

FIG. 1 is a flowchart of a PPG control method according to an embodiment of this application;
FIG. 2 is a schematic diagram of a PPG control system according to an embodiment of this application;
FIG. 3 is a schematic diagram of a back of a smartwatch according to an embodiment of this application;
FIG. 4 is a schematic diagram of a display interface of a smartwatch according to an embodiment of this application;
FIG. 5 is a schematic diagram of a PPG module according to an embodiment of this application; and
FIG. 6 is a schematic diagram of a display interface of a smartwatch according to an embodiment of this application.

### DESCRIPTION OF EMBODIMENTS

To make the objective, technical solutions, and advantages of this application clearer, the technical solutions of this application are clearly and completely described below with reference to specific embodiments of this application and corresponding accompanying drawings. Apparently, the described embodiments are merely some rather than all of embodiments of this application. Based on embodiments of this application, all other embodiments obtained by a person of ordinary skill in the art without creative efforts fall within the protection scope of this application.

Terms used in implementations of this application are merely intended to explain specific embodiments of this application, but are not intended to limit this application.

An operation process of a photoplethysmography (photoplethysmography, PPG) module usually includes: A light-emitting diode (light-emitting diode, LED) in the PPG module emits light; light irradiates human skin and then is reflected; and the reflected light is collected by a photodiode (Photo-Diode, PD) in the PPG module to obtain data of the reflected light.

During operation, operation of the PPG module needs to be controlled based on a configured PPG control scheme (operation strategy), and the PPG control scheme includes an operation mode and operation parameters of each device in the PPG module. For example, the PPG control scheme may include any one or a combination of more of the following:

A luminous frequency of an LED in the PPG module. Light emission of the LED may lead to power consumption, and therefore a higher luminous frequency of the LED indicates higher power consumption of the PPG module.

A luminous intensity of the LED in the PPG module. Light emission of the LED may lead to power consumption, and therefore a higher luminous intensity of the LED indicates higher power consumption of the PPG module.

A reflected-light collection frequency of the PD in the PPG module, that is, a PPG sampling rate. The PPG sampling rate is usually consistent with the luminous frequency of the LED in the PPG module, that is, when the LED in the PPG module emits light, the PD in the PPG module collects reflected light; and the collection of the reflected light by the PD may lead to power consumption, and therefore a higher PPG sampling rate indicates higher power consumption of the PPG module.

An integration time of the PD in the PPG module. When the PD collects the reflected light, a process of obtaining the data of the reflected light is not an instantaneous process. The PD needs to collect data of the reflected light for specific duration, and the data collected in the specific duration needs to undergo integral calculation before effective data of the reflected light can be obtained. The specific duration is referred to as an integration time. The collection of the reflected light by the PD may lead to power consumption, and therefore a longer integration time indicates higher power consumption of the PPG module.

A quantity of groups of the data of the reflected light collected by the PD. When the PPG module is used for measurement, the PD may continuously collect a plurality of groups (for example, 100 groups) of data of the reflected light, and form a curve of the data of the reflected light based on the plurality of groups of data of the reflected light; calculate based on the curve of the data of the reflected light, determine a curve peak (peak searching by using an algorithm), and calculate a measurement result based on the curve peak. The collection of the reflected light by the PD may lead to power consumption, and therefore a larger quantity of groups of data of the reflected light that needs to be collected by the PD indicates higher power consumption of the PPG module.

For PPG measurement, one of the most important technical indicators is measurement accuracy. To ensure the measurement accuracy, impact of a measurement environment on measurement accuracy when a PPG control scheme of the PPG module is configured needs to be considered. For example, when there is a gap between a PPG module of a measuring device and human skin, external light may affect collection by the PD. To ensure that the PD collects accurate data of the reflected light, interference factors of external light needs to be considered when the luminous intensity of the LED in the PPG control scheme is configured. The finally configured luminous intensity of the LED may be higher than that when the interference factors of the external light are not considered.

In an actual measurement scenario of the PPG module, a measurement environment is not unchanging. For example, an intensity of external light may change. In different measurement environments, there are different requirements for PPG performance indicators while measurement accuracy is ensured. To ensure measurement accuracy in different measurement environments, a first measurement environment is usually pre-designed when the PPG control scheme of the PPG module is configured. The first measurement environment is a measurement environment with a highest requirement for a PPG performance indicator in an expected operation scenario of the PPG module. The PPG control scheme of the PPG module (the first PPG control scheme) is configured by referring to the first measurement environment, to ensure that the PPG module can obtain accurate measured data in the first measurement environment. In this way, theoretically, when the PPG module runs in the first PPG control scheme, measurement accuracy can be ensured in all expected operation scenarios. For example, considering impact of external light on collection by the PD, the luminous intensity of the LED is configured by referring to a sunlight intensity at noon. In this way, the luminous intensity of the LED can meet the sunlight intensity in other periods.

Power consumption of the PPG module is different in different PPG control schemes. For example, the power consumption of the LED varies with the configuration of the luminous intensity of the LED in different PPG control schemes. Because the first measurement environment is a measurement environment with a highest requirement for a PPG performance indicator in an expected operation scenario, for a measurement environment with a relatively low performance indicator requirement, when the PPG module runs in the first PPG control scheme, the performance indicator of the PPG module exceeds the performance indicator required to ensure measurement accuracy. In this case, for the purpose of ensuring measurement accuracy, the PPG module has a waste of power.

To avoid a waste of power and reduce the power consumption of the PPG module while ensuring measurement accuracy of the PPG module, this application provides a PPG control method. In the method of this application, a PPG control scheme of the PPG module is determined based on a current measurement environment and a measurement target of a portable device, so that the PPG module can meet a measurement accuracy requirement in the current measurement environment and avoid a waste of power, thereby reducing the power consumption of the PPG module while ensuring the measurement accuracy of the PPG module.

Specifically, the PPG module is installed on the portable device. When the PPG module needs to be enabled for measurement, the portable device detects the current measurement environment and measurement target (a heart rate and/or blood oxygen), invokes a corresponding PPG control scheme based on the current measurement environment, generates a PPG module control instruction based on the invoked PPG control scheme, and sends the PPG module control instruction to the PPG module.

In this embodiment of this application, the portable device may be any electronic device on which a PPG module can be installed. For example, the portable device may be a portable electronic device such as a smartwatch and a smart band. When a user wears the portable device, the PPG module of the portable device is close to the user's skin to implement a measurement operation. For another example, the portable device may alternatively be a non-portable electronic device provided with a PPG module, and the user puts the PPG module of the portable device close to the skin when performing measurement.

Further, the portable device may be an independent electronic device, the independent electronic device includes a data processing module, and measured data collected by the PPG module is processed by the portable device (for example, a smartwatch) to generate a measurement result.

The portable device may alternatively be a part of a measuring system. For example, the portable device does not include a data processing module for processing measured data. A function of the portable device in the measuring system is only to collect measured data. The portable device and another electronic device (for example, a mobile phone) with a data processing module form a measuring system. The user wears a portable device, and after a PPG module of the portable device collects measured data, the measured data is sent to another device in the measuring system for data processing to obtain a measurement result.

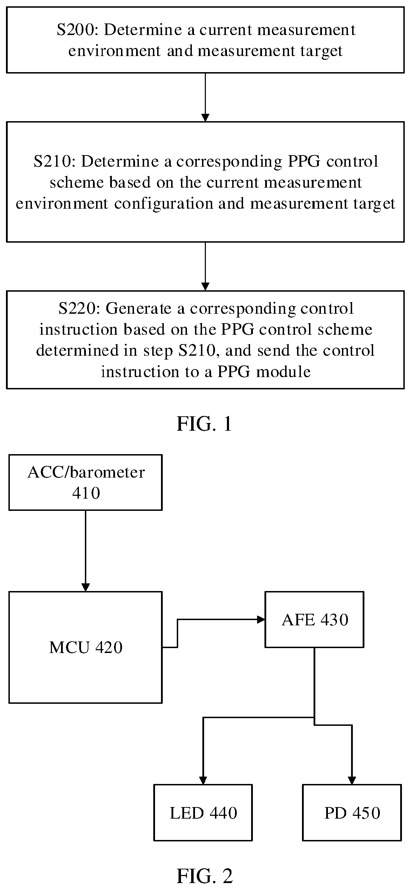

Specifically, FIG. 1 is a flowchart of a PPG control method according to an embodiment of this application. The portable device performs the following steps shown in FIG. 1 to control the PPG module.

S200: Determine a current measurement environment and measurement target, where the measurement target includes measurement of a heart rate and measurement of blood oxygen.

S210: Determine a corresponding PPG control scheme based on the current measurement environment and measurement target.

S220: Generate a corresponding control instruction based on the PPG control scheme determined in step S210, and send the control instruction to the PPG module.

In the method according to this embodiment of this application, different PPG control schemes are used based on different measurement environments and measurement targets, so that a waste of power can be avoided while measurement accuracy is ensured, thereby reducing power consumption of the PPG module while ensuring measurement accuracy of the PPG module.

In an actual application scenario, a person skilled in the art can design a specific implementation of steps S200-S220 based on a specific application requirement, which is not specifically limited in this application. The specific implementation of step S200-S220 is described below by using an example.

In an actual application scenario, many environmental factors affect the PPG module. Therefore, in step S200, one or more measurement environment parameters may be identified to confirm one or more types of characteristics of the measurement environment, so that a PPG control scheme of the PPG module can be determined pertinently subsequently.

Specifically, in an actual application scenario, when the human body is in a motion state, relative positions of the portable device and the human skin are often in a dynamic change state; however, when the human body is in a still state, the relative positions of the portable device and the human skin are often in a still state. Because the PPG module needs to collect light that is emitted by a light emitting device and that is reflected by skin, to ensure measurement accuracy of the PPG module, a requirement for luminous parameters of the light emitting device of the PPG module when the human body is in a motion state is different from that when the human body is in a motion state.

Therefore, in an implementation of step S200, a user state is identified. For example, it is identified whether the user state is a motion state or a non-motion state.

Specifically, in an implementation of step S200, a default user state is a non-motion state, and the user may manually click to enter the motion mode to change the user state to the motion state.

Further, generally, when the user is in a non-motion state, the user may be working, reading, or sleeping. Therefore, to accurately identify the current measurement environment, in another implementation of step S200, during identification of the user state, when the user state is a non-motion state, whether the user is in a sleep state is further identified, to distinguish whether the user is in a rest state or a sleep state. For example, it is identified whether the user state is a motion state, a rest state, or a sleep state.

Further, when the user state is in the motion state, further identification may be performed to distinguish an intensity of the motion, such as large-amplitude intense motion and small-amplitude motion.

Specifically, in an implementation of step S200, the portable device can determine the user state based on measured data of an accelerometer (ACC) and a barometer arranged thereon. For example, by using the built-in ACC (accelerometer) of a watch, motion states of the watch in three directions X, Y, and Z are determined, and with reference to model analysis of the user's motion, whether the user is moving (walking, pace running, and cycling may be judged) is determined after continuous tracking for a period of time (at least 5 minutes). For another example, by using the built-in ACC (accelerometer) of the watch, the motion states of the watch in the three directions X, Y, and Z are determined, and with reference to the user's heart rate data (the heart rate decreases during sleep), whether the user is sleeping is determined after continuous tracking for a period of time (at least 10 minutes).

FIG. 2 is a schematic diagram of a PPG control system according to an embodiment of this application. As shown in FIG. 2, an ACC/barometer 410 collects acceleration/barometric data, and sends a data collection result to a processor MCU 420. The MCU 420 identifies a current measurement environment based on an acceleration/barometric data collection result. The MCU 420 determines a PPG control scheme based on the current measurement environment and measurement target, and generates a corresponding control instruction. The MCU 420 sends the control instruction to a control unit AFE 430 of the PPG module, and the AFE 430 controls a light emitting device LED 440 and a photoelectric conversion device PD 450 of the PPG module based on the received control instruction.

In step S210, a corresponding PPG control scheme may be generated based on the current measurement environment and measurement target; or before step S210, a plurality of PPG control schemes are pre-generated for different measurement environments and/or measurement targets, and in step S210, a PPG control scheme matching the current measurement environment and measurement target is invoked from the pre-generated PPG control schemes.

In an actual application scenario, a person skilled in the art may configure corresponding PPG control schemes for different measurement environments and/or measurement targets based on actual requirements. Specific content of a PPG control scheme is not specifically limited in this application. Specific content of a PPG control scheme is described below by using a specific embodiment as an example.

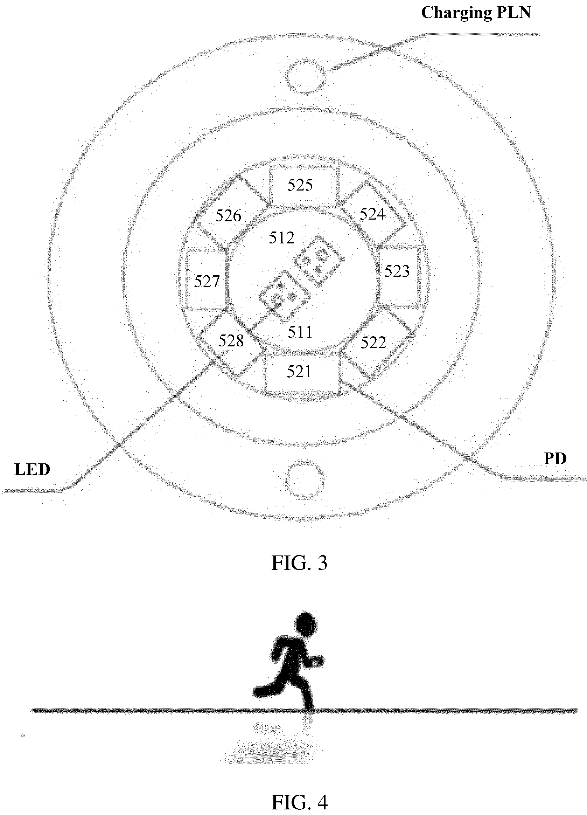

FIG. 3 is a schematic diagram of a back of a smartwatch according to an embodiment of this application. A smartwatch 500 shown in FIG. 3 is provided with a PPG module, and a light emitting device and a photoelectric conversion device of the PPG module are installed on the back of the smartwatch 500.

The light emitting device of the PPG module is an LED 511 and an LED 512. The LED 511 and the LED 512 are both three-in-one LEDs. The LED 511 and the LED 512 can emit green light, red light, and infrared light. The PPG module can control light emitting types of the LED 511 and the LED 512 based on an input control instruction. The photoelectric conversion device of the PPG module is PDs 521-528. A charging PLN is a charging interface of the smartwatch 500.

The smartwatch 500 can identify a current measurement environment (a user state: a motion state, a rest state, or a sleep state) (a motion state identification result is displayed on the smartwatch 500 as shown in FIG. 4) and measurement target (a heart rate or blood oxygen).

The smartwatch 500 may invoke one of a plurality of preconfigured PPG control schemes based on different user states and measurement targets. The smartwatch 500 controls operation of the PPG module based on the invoked PPG control scheme. The following example illustrates a specific implementation in which the smartwatch 500 controls operation of a PPG module based on a current measurement environment and measurement target.

### Embodiment 1

In step S210, a PPG control scheme invoked by the smartwatch 500 includes an enabled light emitting device(s) (an LED 511 and/or an LED 512), a light emitting type of the light emitting device(s) (green light, or red light, or infrared light), a PPG sampling rate, a PPG integration time, and a quantity of groups of data of the reflected light to be collected in each round of measurement.

Specifically, a quantity of light emitting devices enabled in the PPG control scheme invoked by the smartwatch 500 matches a specific user state. When the user state is a motion state, to ensure measurement accuracy, a luminous intensity should be enhanced as far as possible, and the LED 511 and the LED 512 should be enabled to emit light. When the user state is in a rest or sleep state, to reduce power consumption, one of the LED 511 and the LED 512 is enabled to emit light.

It should be noted herein that in this embodiment, different quantities of light emitting devices are enabled based on the user state, and the purpose is to adopt different emitted-light intensities based on the user state, so that a lower emitted-light intensity is adopted when the user state is a rest or sleep state than when the user state is a motion state.

Because the PPG module of the smartwatch 500 is provided with two light emitting devices with a same function, namely the LED 511 and the LED 512, in this embodiment, the quantity of light emitting devices enabled is changed to achieve different emitted-light intensities. In another embodiment of the method according to this application, different emitted-light intensities can be achieved in another manner based on a specific configuration of the light emitting devices of the PPG module. For example, the emitted-light intensity of LEDs is changed by changing a drive voltage/drive current of the LEDs.

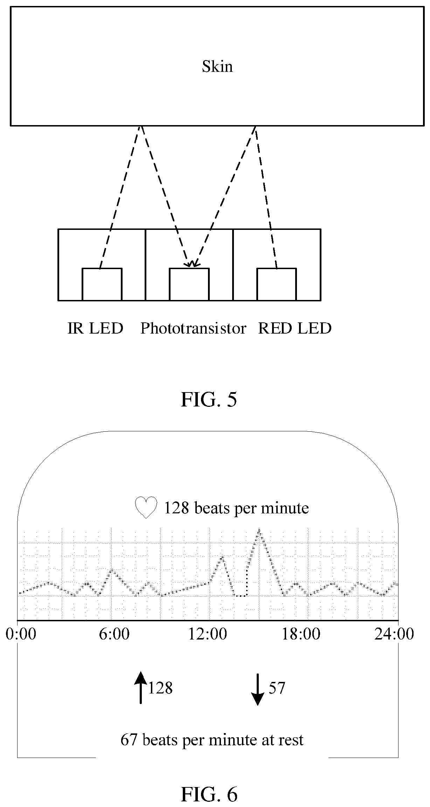

Further, the PP module is usually provided with a red light-emitting diode and an infrared light-emitting diode, so as to use red light and infrared light to measure a heart rate and blood oxygen. For example, FIG. 5 is a schematic diagram of a PPG module according to an embodiment of this application. As shown in FIG. 5, a light emitting/lighting unit of the PPG module includes an infrared light-emitting diode (IR LED), a red light-emitting diode (Red LED), and a phototransistor. Infrared light emitted by the IR LED is collected by the phototransistor after being reflected by skin, and red light emitted by the Red LED is collected by the phototransistor after being reflected by skin.

In an actual application scenario, the PPG module may alternatively measure a heart rate based on light other than red light and infrared light. Specifically, when the heart rate is measured, a better signal is obtained when green light is used as a light source compared with when red light is used. One of reasons is that blood more easily absorbs green light. The blood is red because of hemoglobin in red blood cells. Compared with red light, green light can be absorbed by the hemoglobin. Some red light is absorbed by moisture on the skin, which affects a measurement result. Therefore, when the power consumption is the same, measurement accuracy when green light is used is higher than measurement accuracy when red light is used; that is, when the measurement accuracy is the same, the power consumption when green light is used is lower.

Therefore, in this embodiment, to improve heart rate measurement accuracy and reduce power consumption of emitted light, a luminous type of the light emitting device enabled in the PPG control scheme invoked by the smartwatch 500 matches a measurement target. When the measurement target is a heart rate, the LED 511 and/or the LED 512 emit(s) green light, to improve the measurement accuracy; or when the measurement target is blood oxygen, the LED 511 and/or the LED 512 emit(s) red light and infrared light.

Further, when the measurement target is blood oxygen, the LED(s) of the PPG module need(s) to emit red light and infrared light, and the PD of the PPG module needs to collect data of the reflected light of red light and infrared light separately. Therefore, in this embodiment, when the measurement target is blood oxygen, the LED 511 and/or the LED 512 alternately emit(s) red light and infrared light to avoid mutual interference between the red light and the infrared light, thereby greatly improving accuracy of collecting data of the reflected light. In addition, to ensure accuracy of collecting data of the reflected light, when collecting data of the reflected light, the PD collects data of the reflected light of reflected light of only red light or infrared light at a same time. By adopting a mode of alternately emitting red light and infrared light, an emitted-light collection frequency of the PD matches that of a frequency of alternately emitting red light and infrared light, which can greatly improve effective utilization of LED light.

Further, the PPG sampling rate is a frequency at which the PD measures reflected light during operation of the PPG module. In a motion state, a change frequency of a distance between the PPG module and the user's skin is much greater than that in a non-motion state. To ensure measurement accuracy, the PPG module needs to adopt a higher PPG sampling rate in the motion state than that in a non-motion state.

A higher PPG sampling rate indicates higher power consumption of the PPG module during operation. To reduce power consumption, the PPG sampling rate needs to be kept to match PPG performance parameters required to ensure measurement accuracy, so as to avoid an excessive PPG sampling rate. Therefore, in this embodiment, the PPG sampling rate in the PPG control scheme invoked by the smartwatch 500 matches a specific user state, and the PPG sampling rate for the motion state is greater than that for the non-motion state. When the user state is the motion state, the PPG sampling rate is 100 Hz (first sampling rate); or when the user state is the non-motion state, the PPG sampling rate is 25 Hz (second sampling rate).

It should be noted herein that a specific value of the PPG sampling rate is not specifically limited in this application, and in another embodiment, a person skilled in the art can configure a specific value of the PPG sampling rate based on an actual requirement.

Further, the PPG integration time is a measurement time for the PD to measure reflected light each time during operation of the PPG module. In a motion state, a change frequency of a distance between the PPG module and the user's skin is much greater than that in a non-motion state. To ensure measurement accuracy, the PPG module needs to adopt a longer PPG integration time in the motion state than that in the non-motion state.

A longer PPG integration time indicates higher power consumption of the PPG module during operation. To reduce power consumption, the PPG integration time needs to be kept to match PPG performance parameters required to ensure measurement accuracy, so as to avoid an excessive PPG integration time. Therefore, in this embodiment, the PPG integration time in the PPG control scheme invoked by the smartwatch 500 matches a specific user state, and the PPG integration time for the motion state is greater than that for the non-motion state. When the user state is the motion state, the PPG integration time is 79 us (first integration time); or when the user state is the non-motion state, the PPG integration time is 39 us (second integration time).

It should be noted herein that a specific value of the PPG integration time is not specifically limited in this application, and in another embodiment, a person skilled in the art can configure a specific value of the PPG integration time based on an actual requirement.

Further, in an actual application scenario, when the user is sleeping, if a portable device emits visible light (for example, red light or green light), sleep quality of the user may be affected. Therefore, in this embodiment, a luminous type of light emitting devices in the PPG control scheme invoked by the smartwatch 500 matches a user state. When the user is in a sleep state, during measurement of a heart rate, the LED 511 and/or the LED 512 are/is enabled to emit infrared light; or when the user is in a motion or rest state, during measurement of the heart rate, the LED 511 and/or LED 512 emit(s) green light.

In summary, in Embodiment 1, when the measurement target is a heart rate, and the user state is a motion state, the smartwatch 500 enables the LED 511 and the LED 512 to emit green light. The PDs 521-528 collect 100 groups of data at a sampling rate of 100 Hz for an integration time of 79 us, and then upload the data to a data processing module (a data processing module of the smartwatch 500 or a data processing unit of the PPG module) for peak searching by using an algorithm. A measurement result is displayed on the smartwatch 500 as shown in FIG. 6. In FIG. 6, a current heart rate measurement result of 128 beats per minute is displayed at the top. As the user state is currently a motion state, the smartwatch 500 invokes a heart rate result (67 beats per minute at rest) measured in a rest state in historical records, and the result is displayed at the bottom in FIG. 6.

When the measurement target is a heart rate, and the user state is a rest state, the smartwatch 500 enables the LED 511 or the LED 512 to emit green light (enables one LED). The PDs 521-528 collect 100 groups of data at a sampling rate of 25 Hz for an integration time of 39 us, and then upload the data to the data processing module for peak searching by using an algorithm.

When the measurement target is a heart rate, and the user state is a sleep state, the smartwatch 500 enables the LED 511 or the LED 512 to emit infrared light. The PDs 521-528 collect 100 groups of data at a sampling rate of 25 Hz for an integration time of 39 us, and then upload the data for peak searching by using an algorithm.

When the measurement target is blood oxygen, and the user state is a motion state, the smartwatch 500 enables the LED 511 and the LED 512 to alternately emit red light and infrared light. The PDs 521-528 collect 100 groups of data at a sampling rate of 100 Hz for an integration time of 79 us, and then upload the data to the data processing module for peak searching by using an algorithm.

When the measurement target is blood oxygen, and the user state is a rest or sleep state, the smartwatch 500 enables the LED 511 or the LED 512 to alternately emit red light and infrared light (enables one LED). The PDs 521-528 collect 100 groups of data at a sampling rate of 25 Hz for an integration time of 39 us, and then upload the data to the data processing module for peak searching by using an algorithm.

### Embodiment 2

When the measurement target is a heart rate, and the user state is a motion state, the smartwatch 500 enables the LED 511 and the LED 512 to emit green light. The PDs 521-528 collect 100 groups of data at a sampling rate of 100 Hz for an integration time of 79 us, and then upload the data to a data processing module (a data processing module of the smartwatch 500 or a data processing unit of the PPG module) for peak searching by using an algorithm.

When the measurement target is a heart rate, and the user state is a rest state, the smartwatch 500 enables the LED 511 or the LED 512 to emit green light (enables one LED). The PDs 521-528 collect 100 groups of data at a sampling rate of 25 Hz for an integration time of 79 us, and then upload the data to a data processing module for peak searching by using an algorithm.

When the measurement target is a heart rate, and the user state is a sleep state, the smartwatch 500 enables the LED 511 or the LED 512 to emit infrared light. The PDs 521-528 collect 100 groups of data at a sampling rate of 25 Hz for an integration time of 79 us, and then upload the data for peak searching by using an algorithm.

When the measurement target is blood oxygen, and the user state is a motion state, the smartwatch 500 enables the LED 511 and the LED 512 to alternately emit red light and infrared light. The PDs 521-528 collect 100 groups of data at a sampling rate of 100 Hz for an integration time of 79 us, and then upload the data to the data processing module for peak searching by using an algorithm.

When the measurement target is blood oxygen, and the user state is a rest or sleep state, the smartwatch 500 enables the LED 511 or the LED 512 to alternately emit red light and infrared light (enables one LED). The PDs 521-528 collect 100 groups of data at a sampling rate of 25 Hz for an integration time of 79 us, and then upload the data to a data processing module for peak searching by using an algorithm.

In Embodiment 1, in a PPG control scheme invoked by the smartwatch 500, an enabled light emitting device(s) (an LED 511 and/or an LED 512), a light emitting type of the light emitting device(s) (green light, or red light, or infrared light), a PPG sampling rate, and a PPG integration time match a current measurement environment and measurement target. That is, based on different measurement environments and measurement targets, during operation, the PPG module of the smartwatch 500 enables a corresponding light emitting device and adopts a corresponding luminous type, a corresponding PPG sampling rate, and a corresponding PPG integration time. In addition, based on different measurement environments and measurement targets, the PPG module of the smartwatch 500 adopts a same quantity of groups (100 groups) of data of the reflected light to be collected in each round of measurement.

In Embodiment 2, in a PPG control scheme invoked by the smartwatch 500, an enabled light emitting device(s) (an LED 511 and/or an LED 512), a light emitting type of the light emitting device(s) (green light, or red light, or infrared light), and a PPG sampling rate match a current measurement environment and measurement target. That is, based on different measurement environments and measurement targets, during operation, the PPG module of the smartwatch 500 enables a corresponding light emitting device and adopts a corresponding luminous type and a corresponding PPG sampling rate. In addition, based on different measurement environments and measurement targets, the PPG module of the smartwatch 500 adopts a same PPG integration time (79 us) and a same quantity of groups (100 groups) of data of the reflected light to be collected in each round of measurement.

In another embodiment of this application, a PPG control scheme invoked by the smartwatch 500 may be a configuration scheme different from those in Embodiment 1 and Embodiment 2. For example, based on different measurement environments and measurement targets, the PPG module of the smartwatch 500 adopts different quantities of groups of data of the reflected light to be collected in each round of measurement (when the user state is a motion state, the PPG module collects 100 groups of data, and uploads the data for peak searching by using an algorithm; or when the user state is a rest or sleep state, the PPG module collects 80 groups of data, and uploads the data for peak searching by using an algorithm).

Further, the PPG control method of this application may be applied to another application scenario different from the application scenario shown in FIG. 4. A person skilled in the art can make adaptive adjustments to the PPG control method of this application based on a specific structure of the PPG module, and/or a specific functional configuration of a control device of the PPG module, and/or a specific functional configuration of a measuring system to which a control device of the PPG module belongs. The following uses an example for description in a specific application scenario.

### Embodiment 3

A light emitting device of a PPG module includes an LED 611 and an LED 612, and the LED 611 and the LED 612 are two-in-one LEDs. The LED 611 and the LED 612 can emit red light and infrared light, but cannot emit green light. A control device (for example, a smartwatch) of the PPG module can identify a current measurement environment (a user state: a motion state, a rest state, or a sleep state) and measurement target (a heart rate or blood oxygen).

When the measurement target is a heart rate, and the user state is a motion state, both the LED 611 and the LED 612 are enabled to emit red light. The PDs of the PPG module collect 100 groups of data at a sampling rate of 100 Hz for an integration time of 79 us, and then upload the data to the data processing module for peak searching by using an algorithm.

When the measurement target is a heart rate, and the user state is a rest state, the LED 611 or the LED 612 is enabled to emit red light. The PDs of the PPG module collect 100 groups of data at a sampling rate of 25 Hz for an integration time of 79 us, and then upload the data to the data processing module for peak searching by using an algorithm.

When the measurement target is a heart rate, and the user state is a sleep state, the LED 611 or the LED 612 is enabled to emit infrared light. The PDs of the PPG module collect 100 groups of data at a sampling rate of 25 Hz for an integration time of 79 us, and then upload the data for peak searching by using an algorithm.

When the measurement target is blood oxygen, and the user state is a motion state, both the LED 611 and the LED 612 are enabled to alternately emit red light and infrared light. The PDs of the PPG module collect 100 groups of data at a sampling rate of 100 Hz for an integration time of 79 us, and then upload the data to the data processing module for peak searching by using an algorithm.

When the measurement target is blood oxygen, and the user state is a rest or sleep state, the LED 611 or the LED 612 is enabled to alternately emit red light and infrared light (one LED is enabled). The PDs of the PPG module collect 100 groups of data at a sampling rate of 25 Hz for an integration time of 79 us, and then upload the data to the data processing module for peak searching by using an algorithm.

### Embodiment 4

A light emitting device of a PPG module includes only an LED 711, and the LED 711 is a two-in-one LED. The LED 711 can emit red light and infrared light, but cannot emit green light.

When a measurement target is a heart rate, and a user state is a motion state, the LED 711 is enabled to emit red light. The PDs of the PPG module collect 100 groups of data at a sampling rate of 100 Hz for an integration time of 79 us, and then upload the data to the data processing module for peak searching by using an algorithm.

When the measurement target is a heart rate, and the user state is a rest state, the LED 711 is enabled to emit red light. The PDs of the PPG module collect 100 groups of data at a sampling rate of 25 Hz for an integration time of 79 us, and then upload the data to the data processing module for peak searching by using an algorithm.

When the measurement target is a heart rate, and the user state is a sleep state, the LED 711 is enabled to emit infrared light. The PDs of the PPG module collect 100 groups of data at a sampling rate of 25 Hz for an integration time of 79 us, and then upload the data for peak searching by using an algorithm.

When the measurement target is blood oxygen, and the user state is a motion state, the LED 711 is enabled to alternately emit red light and infrared light. The PDs of the PPG module collect 100 groups of data at a sampling rate of 100 Hz for an integration time of 79 us, and then upload the data to the data processing module for peak searching by using an algorithm.

When the measurement target is blood oxygen, and the user state is a rest or sleep state, the LED 711 is enabled to alternately emit red light and infrared light (one LED is enabled). The PDs of the PPG module collect 100 groups of data at a sampling rate of 25 Hz for an integration time of 79 us, and then upload the data to the data processing module for peak searching by using an algorithm.

### Embodiment 5

A light emitting device of the PPG module includes an LED 811 and an LED 812. The LED 811 and the LED 812 are both three-in-one LEDs. The LED 811 and the LED 812 can emit green light, red light, and infrared light. A control device (for example, a smartwatch) of the PPG module can identify a current measurement environment (a user state: a motion state or a non-motion state) and measurement target (a heart rate or blood oxygen). However, the control device (for example, the smartwatch) of the PPG module cannot further identify the non-motion state, and cannot distinguish a rest state from a sleep state.

When the measurement target is a heart rate, and the user state is a motion state, both the LED 811 and the LED 812 are enabled to emit green light. The PDs of the PPG module collect 100 groups of data at a sampling rate of 100 Hz for an integration time of 79 us, and then upload the data to the data processing module for peak searching by using an algorithm.

When the measurement target is a heart rate, and the user state is a non-motion state, the LED 811 or the LED 812 is enabled to emit green light. The PDs of the PPG module collect 100 groups of data at a sampling rate of 25 Hz for an integration time of 79 us, and then upload the data to the data processing module for peak searching by using an algorithm.

When the measurement target is blood oxygen, and the user state is a motion state, both the LED 811 and the LED 812 are enabled to alternately emit red light and infrared light. The PDs of the PPG module collect 100 groups of data at a sampling rate of 100 Hz for an integration time of 79 us, and then upload the data to the data processing module for peak searching by using an algorithm.

When the measurement target is blood oxygen, and the user state is a non-motion state, the LED 811 or the LED 812 is enabled to alternately emit red light and infrared light (one LED is enabled). The PDs of the PPG module collect 100 groups of data at a sampling rate of 25 Hz for an integration time of 79 us, and then upload the data to the data processing module for peak searching by using an algorithm.

According to the PPG control method in this application, this application further provides a PPG control apparatus. The apparatus is applied to an electronic device (for example, a smartwatch), the electronic device includes a PPG module (for example, a PPG module with a structure shown in FIG. 3 or FIG. 5), and the PPG module includes a light emitting module. The apparatus includes:
a collection module, configured to determine a current measurement environment and/or measurement target, where the measurement environment includes motion, rest, and sleep, and the measurement target includes a heart rate and blood oxygen; and
a control module, configured to determine a light emitting mode of the light emitting module based on the current measurement environment and/or measurement target.

In the description of this embodiment of this application, for ease of description, the apparatus is divided into various modules based on functions for description, and the division of the modules is only a division of logical functions. When this embodiment of this application is implemented, the functions of the modules may be implemented in one or more pieces of software and/or hardware.

Specifically, in actual implementation, the apparatus in this embodiment of this application can be fully or partially integrated into a physical entity, or may be physically separated. In addition, all the modules may be implemented by software invoked by a processing element, or may be implemented by hardware; or some modules may be implemented by software invoked by a processing element, and some modules are implemented by hardware. For example, a detection module may be a separate processing element, or may be integrated into a chip of the electronic device for implementation. The implementation of another module is similar thereto. In addition, all or some of these modules may be integrated together, or may be implemented independently. In an implementation process, the steps in the foregoing methods or the foregoing modules may be implemented by using an integrated logic circuit of hardware in the processing element or an instruction in a form of software.

For example, these foregoing modules may be one or more integrated circuits configured to implement the foregoing method, such as one or more application specific integrated circuits (Application Specific Integrated Circuit, ASIC), one or more digital signal processors (Digital Signal Processor, DSP), or one or more field programmable gate arrays (Field Programmable Gate Array, FPGA). For another example, these modules may be integrated together, and implemented in a form of a system-on-a-chip (System-On-a-Chip, SOC).

An embodiment of this application further provides an electronic device (for example, a smartwatch). The electronic device includes a memory configured to store a computer program instruction and a processor configured to execute the program instruction. When the computer program instruction is executed by the processor, the electronic device is triggered to perform the steps of the method according to the embodiments of this application.

Specifically, in an embodiment of this application, the foregoing one or more computer programs are stored in the foregoing memory, the one or more computer programs include instructions, and when the foregoing instructions are executed by the foregoing device, the foregoing device is enabled to perform the steps of the method according to the embodiments of this application.

Specifically, in an embodiment of this application, the processor of the electronic device may be a system-on-a-chip SOC, and the processor may include a central processing unit (Central Processing Unit, CPU), and may further include another type of processors. Specifically, in an embodiment of this application, the processor of the electronic device may be a PWM control chip.

Specifically, in an embodiment of this application, the involved processor may include, for example, a CPU, a DSP, a microcontroller, or a digital signal processor, and may further include a GPU, a neural-network process unit (Neural-network Process Units, NPU), and an image signal processor (Image Signal Processing, ISP). The processor may further include a necessary hardware accelerator or logic processing hardware circuit, such as an ASIC, one or more integrated circuits used to control execution of a technical solution program in this application, or the like. In addition, the processor may have a function of operating one or more software programs, and the software programs may be stored in a storage medium.

Specifically, in an embodiment of this application, the memory of the electronic device may be a read-only memory (read-only memory, ROM), another type of static storage device that can store static information and instructions, a random access memory (random access memory, RAM), or another type of dynamic storage device that can store information and instructions, or may be an electrically erasable programmable read-only memory (electrically erasable programmable read-only memory, EEPROM), a compact disc read-only memory (compact disc read-only memory, CD-ROM) or another optical disk storage, an optical disc storage (including a compact disc, a laser disc, an optical disc, a digital versatile disc, a Blu-ray disc, or the like), a magnetic disk storage medium, or another magnetic storage device, or may be any computer-readable medium that can be used to carry or store expected program code in a form of an instruction or a data structure and that can be accessed by a computer.

Specifically, in an embodiment of this application, the processor and the memory may be combined into a processing apparatus. More commonly, they are separate components. The processor is configured to execute program code stored in the memory to implement the method according to the embodiments of this application. In specific implementation, the memory may be integrated in the processor, or may be independent of the processor.

Further, the device, apparatus, and modules illustrated in embodiments of this application may be specifically implemented by a computer chip or entity, or may be implemented by a product with a specific function.

A person skilled in the art should understand that embodiments of this application may be provided as a method, a system, or a computer program product. Therefore, the present invention can use a form of hardware only embodiments, software only embodiments, or embodiments with a combination of software and hardware. In addition, the present invention can use a form of a computer program product that is implemented on one or more computer-usable storage media that include computer-usable program code.

In several embodiments in this application, if any function is implemented in the form of a software functional unit and sold or used as an independent product, the function may be stored in a computer-readable storage medium. Based on such an understanding, the technical solutions of this application essentially, or the part contributing to the conventional technology, or some of the technical solutions may be implemented in a form of a software product. The software product is stored in a storage medium, and includes several instructions for instructing a computer device (which may be a personal computer, a server, or a network device) to perform all or some of the steps of the methods described in embodiments of this application.

Specifically, an embodiment of this application further provides a computer-readable storage medium, where the computer program is stored in the computer-readable storage medium, and when the computer program runs on a computer, the computer is enabled to perform the method according to the embodiments of this application.

An embodiment of this application further provides a computer program product, where the computer program product includes a computer program, and when the computer program is run on a computer, the computer is enabled to perform the method according to the embodiments of this application.

Embodiments of this application are described with reference to the flowcharts and/or block diagrams of the method, the device (apparatus), and the computer program product according to embodiments of this application. It should be understood that computer program instructions can be used to implement each process and/or each block in the flowcharts and/or the block diagrams and a combination of a process and/or a block in the flowcharts and/or the block diagrams. These computer program instructions can be provided for a general-purpose computer, a special-purpose computer, an embedded processor, or a processor of another programmable data processing device to generate a machine, so that the instructions executed by the computer or the processor of the another programmable data processing device generate an apparatus for implementing a specific function in one or more processes in the flowcharts and/or in one or more blocks in the block diagrams.

These computer program instructions can be stored in a computer-readable memory that can instruct the computer or the another programmable data processing device to operate in a specific way, so that the instructions stored in the computer-readable memory generate an artifact that includes an instruction apparatus. The instruction apparatus implements a specific function in one or more processes in the flowcharts and/or in one or more blocks in the block diagrams.

These computer program instructions can alternatively be loaded onto the computer or another programmable data processing device, so that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

It should also be noted that in embodiments of this application, "at least one" means one or more, and "a plurality of" means two or more. The term "and/or" describes an association relationship of associated objects, and indicates that three relationships may exist. For example, A and/or B may indicate the following cases: Only A exists, both A and B exist, and only B exists, where A and B may be singular or plural. The character "/" usually indicates an "or" relationship between the associated objects. "At least one of the following" or a similar expression thereof indicates any combination of the following, and includes any combination of one or more of the following. For example, at least one of a, b, or c may indicate a, b, c, a and b, a and c, b and c, or a and c, where a, b, and c may indicate a singular or plural form.

In embodiments of this application, the term "including", "containing" or any other variant thereof is intended to cover non-exclusive inclusion, so that a process, method, commodity, or device including a series of elements includes not only those elements, but also other elements not explicitly listed, or elements inherent to such a process, method, commodity, or device. Without further limitation, the element defined by the sentence "including a..." does not exclude that other identical elements are also present in the process, method, commodity, or device including the element.

This application may be described in the general context of computer-executable instructions, such as a program module, executed by a computer. Generally, the program module includes a routine, a program, an object, a component, a data structure, and the like that perform a specific task or implement a specific abstract data type. This application may alternatively be practiced in distributed computing environments, and in these distributed computing environments, a task is performed by a remote processing device connected by using a communication network. In a distributed computing environment, program modules may be located in local and remote computer storage media including storage devices.

All embodiments of this application are described in a progressive way, and for the same and similar parts of embodiments, reference may be made to each other. Each embodiment focuses on differences from other embodiments. Especially, because the apparatus embodiment is substantially similar to the method embodiment, the description is relatively simple. For related parts, refer to the description of the method embodiment.

A person of ordinary skill in the art may be aware that the units and algorithm steps described in embodiments of this specification can be implemented by electronic hardware or a combination of computer software and electronic hardware. Whether these functions are implemented by using hardware or software depends on specific application of the technical solution and design constraints. A person skilled in the art may use different methods to implement the described functions for each particular application, but it should not be considered that the implementation goes beyond the scope of this application.

A person skilled in the art can clearly understand that, for ease and brevity of description, reference may be made to corresponding processes in the foregoing method embodiment for specific operating processes of the foregoing apparatus, apparatus, and unit. Details are not described herein again.

The foregoing descriptions are merely specific implementations of this application. Any person skilled in the art can easily figure out modifications or replacements within the technical scope disclosed in this application, and these modifications or replacements shall fall within the protection scope of this application. The protection scope of this application shall be subject to the protection scope of the claims.

## Claims

1. A photoplethysmography, PPG, control method, wherein the method is applied to an electronic device, the electronic device comprises a PPG module, the PPG module comprises a light emitting module and a photodiode to collect the reflected light, and the method comprises:
determining (S200) a current measurement environment and measurement target, wherein the measurement environment comprises at least one of motion, rest, and sleep, and the measurement target comprises at least one of a heart rate and blood oxygen; and
determining (S210) a light emitting mode of the light emitting module either based on the current measurement environment or based on the current measurement environment and the measurement target; and **characterised in that** the method further comprises:
determining an integration time of the PPG module based on the current measurement environment, and
using a first integration time when the measurement environment is motion; or using a second integration time when the measurement environment is rest or sleep, wherein the first integration time is greater than the second integration time; and
wherein using the integration time comprises collecting data of the reflected light for the integration time, the data collected undergoing integral calculation to obtain effective data of the reflected light.

2. The method according to claim 1, wherein the light emitting module comprises a plurality of light emitting devices (440), and the plurality of light emitting devices (440) support emission of a same type of light; and
the determining (S210) a light emitting mode of the light emitting module based on the current measurement environment comprises:
determining (S210) the light emitting mode of the light emitting module based on the measurement environment, and enabling (S220) all light emitting devices (440) of the light emitting module when the measurement environment is motion; or enabling (S220) some light emitting devices (440) of the light emitting module when the measurement environment is rest or sleep.

3. The method according to claim 1, wherein the light emitting module comprises a fourth light emitting device (440) and a fifth light emitting device (440), the fourth light emitting device (440) is configured to emit visible light, and the fifth light emitting device (440) is configured to emit infrared light; and
the determining (S210) a light emitting mode of the light emitting module based on the current measurement environment comprises:
determining (S210) the light emitting mode of the light emitting module based on the measurement environment, and enabling (S220) the fourth light emitting device (440) when the measurement environment is motion or rest; or enabling (S220) the fifth light emitting device (440) when the measurement environment is sleep.

4. The method according to claim 1, wherein the light emitting module comprises an eighth light emitting device (440), a ninth light emitting device (440), a tenth light emitting device (440), and an eleventh light emitting device (440), wherein the eighth light emitting device (440) and the ninth light emitting device (440) are configured to emit green light, and the tenth light emitting device (440) and the eleventh light emitting device (440) are configured to emit red light and infrared light; and
the determining (S210) a light emitting mode of the light emitting module based on the current measurement environment and measurement target comprises:
enabling (S220) the eighth light emitting device (440) and the ninth light emitting device (440) when the measurement environment is motion, and the measurement target is a heart rate; or
enabling (S220) the eighth light emitting device (440) or the ninth light emitting device (440) when the measurement environment is stillness, and the measurement target is a heart rate; or
enabling (S220) the tenth light emitting device (440) or the eleventh light emitting device (440) to emit infrared light when the measurement environment is sleep, and the measurement target is a heart rate; or
enabling (S220) the tenth light emitting device (440) and the eleventh light emitting device (440) to alternately emit red light and infrared light when the measurement environment is motion, and the measurement target is blood oxygen; or
enabling (S220) the tenth light emitting device (440) or the eleventh light emitting device (440) to alternately emit red light and infrared light when the measurement environment is stillness or sleep, and the measurement target is blood oxygen.

5. The method according to any one of claims 1 to 4, wherein the method further comprises:
determining a sampling rate of the PPG module based on the current measurement environment, and
using a first sampling rate when the measurement environment is motion; or using a second sampling rate when the measurement environment is rest or sleep, wherein the first sampling rate is greater than the second sampling rate.

6. The method according to claim 5, wherein the first sampling rate is 100 Hz, and the second sampling rate is 25 Hz.

7. The method according to claim 1, wherein the first integration time is 79 us, and the second integration time is 39 us.

8. An electronic device, wherein the electronic device comprises a PPG module, the PPG module comprises a light emitting module and a photodiode to collect the reflected light, a memory configured to store a computer program instruction and a processor configured to execute a computer program instruction, and when the computer program instruction is executed by the processor, the electronic device is triggered to perform the steps of the method according to any one of claims 1 to 7.

9. A computer program product, comprising instructions to cause the device of claim 8 to execute the steps of the method according to any one of claims 1 to 7.

10. A computer-readable storage medium, wherein the computer-readable storage medium having stored thereon the computer program product of claim 9.

## Patentansprüche

1. Ein Steuerungsverfahren für die Photoplethysmographie (PPG), wobei das Verfahren auf ein elektronisches Gerät angewendet wird, das elektronische Gerät ein PPG-Modul umfasst, das PPG-Modul ein Lichtemissionsmodul und eine Photodiode zur Sammlung des reflektierten Lichts umfasst, und das Verfahren Folgendes umfasst:
Bestimmung (S200) einer aktuellen Messumgebung und eines Messziels, wobei die Messumgebung mindestens eine der folgenden Bedingungen umfasst: Bewegung, Ruhe und Schlaf, und das Messziel mindestens eine der folgenden Bedingungen umfasst: Herzfrequenz und Blutsauerstoff; und
Bestimmung (S210) eines Lichtemissionsmodus des Lichtemissionsmoduls entweder basierend auf der aktuellen Messumgebung oder basierend auf der aktuellen Messumgebung und dem Messziel; und **dadurch gekennzeichnet, dass** das Verfahren weiterhin Folgendes umfasst:
Bestimmung einer Integrationszeit des PPG-Moduls basierend auf der aktuellen Messumgebung; und
Verwendung einer ersten Integrationszeit, wenn die Messumgebung Bewegung ist; oder Verwendung einer zweiten Integrationszeit, wenn die Messumgebung Ruhe oder Schlaf ist, wobei die erste Integrationszeit größer ist als die zweite Integrationszeit; und
wobei die Verwendung der Integrationszeit das Sammeln von Daten des reflektierten Lichts für die Integrationszeit umfasst, wobei die gesammelten Daten einer Integrationsberechnung unterzogen werden, um die effektiven Daten des reflektierten Lichts zu erhalten.

2. Verfahren nach Anspruch 1, wobei das Lichtemissionsmodul eine Vielzahl von Lichtemissionsvorrichtungen (440) umfasst und die Vielzahl von Lichtemissionsvorrichtungen (440) die Emission des gleichen Lichttyps unterstützen; und
das Bestimmen (S210) eines Lichtemissionsmodus des Lichtemissionsmoduls basierend auf der aktuellen Messumgebung umfasst:
Bestimmen (S210) des Lichtemissionsmodus des Lichtemissionsmoduls basierend auf der Messumgebung und Aktivieren (S220) aller Lichtemissionsvorrichtungen (440) des Lichtemissionsmoduls, wenn die Messumgebung Bewegung ist; oder Aktivieren (S220) einiger Lichtemissionsvorrichtungen (440) des Lichtemissionsmoduls, wenn die Messumgebung Ruhe oder Schlaf ist.

3. Verfahren nach Anspruch 1, wobei das Lichtemissionsmodul eine vierte Lichtemissionsvorrichtung (440) und eine fünfte Lichtemissionsvorrichtung (440) umfasst, die vierte Lichtemissionsvorrichtung (440) ist konfiguriert, sichtbares Licht zu emittieren, und die fünfte Lichtemissionsvorrichtung (440) ist konfiguriert, Infrarotlicht auszusenden; und
das Bestimmen (S210) eines Lichtemissionsmodus des Lichtemissionsmoduls basierend auf der aktuellen Messumgebung umfasst:
Bestimmen (S210) des Lichtemissionsmodus des Lichtemissionsmoduls basierend auf der Messumgebung und Aktivieren (S220) der vierten Lichteinheit (440), wenn die Messumgebung Bewegung oder Ruhe ist; oder Aktivieren (S220) der fünften Lichteinheit (440), wenn die Messumgebung Schlaf ist.

4. Verfahren gemäß Anspruch 1, wobei das Lichtemissionsmodul eine achte Lichteinheit (440), eine neunte Lichteinheit (440), eine zehnte Lichteinheit (440) und eine elfte Lichteinheit (440) umfasst, wobei die achte Lichteinheit (440) und die neunte Lichteinheit (440) so konfiguriert sind, dass sie grünes Licht aussenden, und die zehnte Lichteinheit (440) und die elfte Lichteinheit (440) so konfiguriert sind, dass sie rotes Licht und Infrarotlicht aussenden; und
das Bestimmen (S210) eines Lichtemissionsmodus des Lichtemissionsmoduls basierend auf der aktuellen Messumgebung und dem Messziel umfasst:
Aktivieren (S220) der achten Lichteinheit (440) und der neunten Lichteinheit (440), wenn die Messumgebung Bewegung ist und das Messziel die Herzfrequenz ist; oder
Aktivieren (S220) der achten Lichteinheit (440) oder der neunten Lichteinheit (440), wenn die Messumgebung Stillstand ist und das Messziel die Herzfrequenz ist; oder
Aktivieren (S220) der zehnten Lichteinheit (440) oder der elften Lichteinheit (440) zum Aussenden von Infrarotlicht, wenn die Messumgebung Schlaf ist und das Messziel die Herzfrequenz ist; oder
Aktivieren (S220) der zehnten Lichteinheit (440) und der elften Lichteinheit (440) zum alternierenden Aussenden von rotem Licht und Infrarotlicht, wenn die Messumgebung Bewegung ist und das Messziel Blutsauerstoff ist; oder
Aktivieren (S220) der zehnten Lichteinheit (440) oder der elften Lichteinheit (440) zum alternierenden Aussenden von rotem Licht und Infrarotlicht, wenn die Messumgebung Stillstand oder Schlaf ist und das Messziel Blutsauerstoff ist.

5. Verfahren gemäß einem der Ansprüche 1 bis 4, wobei das Verfahren weiterhin umfasst:
Bestimmen einer Abtastrate des PPG-Moduls basierend auf der aktuellen Messumgebung, und
Verwendung einer ersten Abtastrate, wenn die Messumgebung Bewegung ist; oder Verwendung einer zweiten Abtastrate, wenn die Messumgebung Ruhe oder Schlaf ist, wobei die erste Abtastrate größer als die zweite Abtastrate ist.

6. Verfahren gemäß Anspruch 5, wobei die erste Abtastrate 100 Hz beträgt und die zweite Abtastrate 25 Hz beträgt.

7. Verfahren nach Anspruch 1, wobei die erste Integrationszeit 79 µs beträgt und die zweite Integrationszeit 39 µs beträgt.

8. Elektronisches Gerät, wobei das elektronische Gerät ein PPG-Modul umfasst, wobei das PPG-Modul ein Lichtemissionsmodul und eine Photodiode zum Sammeln des reflektierten Lichts umfasst, einen Speicher, der zum Speichern einer Computerprogrammanweisung konfiguriert ist, und einen Prozessor, der zum Ausführen einer Computerprogrammanweisung konfiguriert ist, und wenn die Computerprogrammanweisung vom Prozessor ausgeführt wird, das elektronische Gerät ausgelöst wird, um die Schritte des Verfahrens nach einem der Ansprüche 1 bis 7 auszuführen.

9. Computerprogrammprodukt, das Anweisungen umfasst, um das Gerät gemäß Anspruch 8 dazu zu veranlassen, die Schritte des Verfahrens nach einem der Ansprüche 1 bis 7 auszuführen.

10. Computerlesbares Speichermedium, wobei das computerlesbare Speichermedium das Computerprogrammprodukt nach Anspruch 9 darauf gespeichert enthält.

## Revendications

1. Procédé de contrôle de photopléthysmographie (PPG), dans lequel le procédé est appliqué à un dispositif électronique, le dispositif électronique comprenant un module PPG, le module PPG comprenant un module d'émission de lumière et une photodiode pour collecter la lumière réfléchie, et le procédé comprend :
détermination (S200) d'un environnement de mesure actuel et d'une cible de mesure, l'environnement de mesure comprenant au moins l'un des éléments suivants : mouvement, repos et sommeil, et la cible de mesure comprenant au moins l'un des éléments suivants : fréquence cardiaque et oxygène sanguin ; et
détermination (S210) d'un mode d'émission du module d'émission de lumière soit en fonction de l'environnement de mesure actuel, soit en fonction de l'environnement de mesure actuel et de la cible de mesure ; et **caractérisé en ce que** le procédé comprend en outre :
détermination d'un temps d'intégration du module PPG sur la base de l'environnement de mesure actuel, et
utilisation d'un premier temps d'intégration lorsque l'environnement de mesure est le mouvement ; ou utilisation d'un second temps d'intégration lorsque l'environnement de mesure est le repos ou le sommeil, le premier temps d'intégration étant supérieur au second temps d'intégration ; et
dans lequel l'utilisation du temps d'intégration consiste à collecter les données de la lumière réfléchie pendant le temps d'intégration, les données collectées subissant un calcul intégral afin d'obtenir des données effectives de la lumière réfléchie.

2. Procédé selon la revendication 1, dans lequel le module d'émission de lumière comprend une pluralité de dispositifs d'émission de lumière (440), et la pluralité de dispositifs d'émission de lumière (440) permet l'émission d'un même type de lumière ; et
la détermination (S210) d'un mode d'émission du module d'émission de lumière sur la base de l'environnement de mesure actuel comprend :
détermination (S210) du mode d'émission du module d'émission de lumière sur la base de l'environnement de mesure, et activation (S220) de tous les dispositifs d'émission de lumière (440) du module d'émission de lumière lorsque l'environnement de mesure est le mouvement; ou activation (S220) de certains dispositifs d'émission de lumière (440) du module d'émission de lumière lorsque l'environnement de mesure est le repos ou le sommeil.

3. Procédé selon la revendication 1, dans lequel le module d'émission de lumière comprend un quatrième dispositif d'émission de lumière (440) et un cinquième dispositif d'émission de lumière (440), le quatrième dispositif d'émission de lumière (440) étant configuré pour émettre une lumière visible, et le cinquième dispositif d'émission de lumière (440) étant configuré pour émettre une lumière infrarouge ; et
la détermination (S210) d'un mode d'émission du module d'émission de lumière sur la base de l'environnement de mesure actuel comprend :
déterminer (S210) le mode d'émission de lumière du module d'émission de lumière en fonction de l'environnement de mesure, et activer (S220) le quatrième dispositif d'émission de lumière (440) lorsque l'environnement de mesure est en mouvement ou au repos; ou activer (S220) le cinquième dispositif d'émission de lumière (440) lorsque l'environnement de mesure est le sommeil.

4. La méthode selon la revendication 1, dans laquelle le module d'émission de lumière comprend un huitième dispositif d'émission de lumière (440), un neuvième dispositif d'émission de lumière (440), un dixième dispositif d'émission de lumière (440) et un onzième dispositif d'émission de lumière (440), dans laquelle le huitième dispositif d'émission de lumière (440) et le neuvième dispositif d'émission de lumière (440) sont configurés pour émettre une lumière verte, et le dixième dispositif d'émission de lumière (440) et l'onzième dispositif d'émission de lumière (440) sont configurés pour émettre une lumière rouge et une lumière infrarouge; et
la détermination (S210) d'un mode d'émission de lumière du module d'émission de lumière en fonction de l'environnement de mesure actuel et de la cible de mesure comprend :
activer (S220) le huitième dispositif d'émission de lumière (440) et le neuvième dispositif d'émission de lumière (440) lorsque l'environnement de mesure est en mouvement et que la cible de mesure est la fréquence cardiaque ; ou
activer (S220) le huitième dispositif d'émission de lumière (440) ou le neuvième dispositif d'émission de lumière (440) lorsque l'environnement de mesure est au repos, et que la cible de mesure est la fréquence cardiaque; ou
activer (S220) le dixième dispositif d'émission de lumière (440) ou l'onzième dispositif d'émission de lumière (440) pour émettre une lumière infrarouge lorsque l'environnement de mesure est le sommeil, et que la cible de mesure est la fréquence cardiaque; ou
activer (S220) le dixième dispositif d'émission de lumière (440) et l'onzième dispositif d'émission de lumière (440) pour émettre en alternance une lumière rouge et une lumière infrarouge lorsque l'environnement de mesure est en mouvement, et que la cible de mesure est l'oxygène sanguin; ou
activer (S220) le dixième dispositif d'émission de lumière (440) ou l'onzième dispositif d'émission de lumière (440) pour émettre en alternance une lumière rouge et une lumière infrarouge lorsque l'environnement de mesure est au repos ou pendant le sommeil, et que la cible de mesure est l'oxygène sanguin.

5. La méthode selon l'une quelconque des revendications 1 à 4, dans laquelle la méthode comprend en outre:
déterminer une fréquence d'échantillonnage du module PPG en fonction de l'environnement de mesure actuel, et
utiliser une première fréquence d'échantillonnage lorsque l'environnement de mesure est en mouvement ; ou utiliser une deuxième fréquence d'échantillonnage lorsque l'environnement de mesure est au repos ou pendant le sommeil, la première fréquence d'échantillonnage étant supérieure à la deuxième fréquence d'échantillonnage.

6. La méthode selon la revendication 5, dans laquelle la première fréquence d'échantillonnage est de 100 Hz, et la deuxième fréquence d'échantillonnage est de 25 Hz.

7. Procédé selon la revendication 1, dans lequel le premier temps d'intégration est de 79 µs et le second temps d'intégration est de 39 µs.

8. Dispositif électronique, ledit dispositif électronique comprenant un module PPG, le module PPG comprenant un module d'émission de lumière et une photodiode pour collecter la lumière réfléchie, une mémoire configurée pour stocker une instruction de programme informatique et un processeur configuré pour exécuter une instruction de programme informatique, et lorsque l'instruction de programme informatique est exécutée par le processeur, le dispositif électronique est déclenché pour effectuer les étapes du procédé selon l'une quelconque des revendications 1 à 7.

9. Produit programme informatique, comprenant des instructions pour amener le dispositif selon la revendication 8 à exécuter les étapes du procédé selon l'une quelconque des revendications 1 à 7.

10. Support de stockage lisible par ordinateur, ledit support de stockage lisible par ordinateur stockant le produit programme informatique selon la revendication 9.
